# EUROPEAN PATENT APPLICATION

(11) **EP 1 891 977 A2**
(43) Date of publication of application: **27.02.2008**
(21) Application number: 07012990.3
(22) Date of filing: 03.07.2007
(51) Int. Cl.: A61L 2/04

(54) **Bottled water cooler with hot water sterilization system**

(30) Priority: 03.07.2006 CN 200620105324 U
(71) Applicant: Yui, George, Beilum/Nihgbo Zhejiang 315800 (CN)
(72) Inventor: Yui, George, Beilum/Nihgbo Zhejiang 315800 (CN)
(74) Representative: Marchau, Michel F.L.A.

(57) **Abstract**

The present invention is a bottled water cooler with a hot water sterilization system. A bottled water cooler with a hot water sterilizing system having a cold tank installed on the top of the cooler there is bottle receptacle in it, and the bottle receptacle groove having a piercing core connecting with water bottle, the cold tank inside, there is a baffle through hollow baffle leader under the piercing core, the end of the baffle leader connect with a hot tank installed under cold tank by cold and hot tank connecting tube, hot tank draining three-way pipe or draining pipe. For sterilizing the cool water tank a valve in the conduit between cool and hot water tanks is opened that the hot water flows into the cold water tank due to natural convection without employing any pumping means. A reflow to the inserted bottled is avoided by the provision of a check valve above the cool water tank adjacent to the bottle's neck. The check valve comprises a plate-shaped float which is movable on a rail.

## Description

This application claims the benefit of Chinese Patent Application No. 200620105324.2 filed July 3, 2006.

### TECHNICAL FIELD & BACKGROUND

The present invention generally relates to the field of bottled water coolers, more specifically a kind of bottled water cooler with a hot water sterilization system, mainly used for home or office.

Presently, most of the coolers in the market do not have sterilization system; it is hard to avoid bacteria increasing inside of the cooler after being used for a long time making the water polluted and harmful to a user's health. Others invented water coolers with sterilization system, for example Chinese patent No. 200420081793.6, which is a kind of bottled water cooler with ozone sterilizing system, containing a water bottle, a cold water tank, its character is that the water cooler has ozone sterilizing system, which includes an ozonator set up outside of the cold water tank, and ozone emanated equipment set up on the bottom inside of the cold water tank, they are connected by flexible connecting tube. This system also includes an active carbon tank that is connect with the cold water tank lumen. But this kind of cooler needs to install an ozonator and ozone emanated equipment in the common cooler, it is not only complicated in structure, also difficult to install, increasing production cost.

The present invention is a bottled water cooler with a hot water sterilizing system its structure is simple allowing the present invention to be cleaned less often, the present invention can sterilize by water convection in the cold and hot tank lowing the increase in bacteria and ultimately preventing bacteria.

The present invention is a bottled water cooler with a hot water sterilizing system having a cold tank installed on the top of the cooler there is bottle receptacle in it, and the stated bottle receptacle groove having a piercing core connecting with water bottle, the stated cold tank inside, there is a baffle through hollow baffle leader under the piercing core, the end of the baffle leader connect with hot tank installed under cold tank by cold and hot tank connecting tube, hot tank draining three-way pipe or draining pipe. They set up a baffle for cold tank between cold and hot tank. The hot tank has a hot tank outlet and a hot tank exhausting tube between the cold tank and hot tank. There is a cold and hot tank straightly connecting tube with a valve on the top. Inside of the probe is a check floating seal fitting.

The check floating seal fitting is made up of floating board, restricting bolts and mat piece which can prevent floating board from falling off. The floating board is thin disk shape and there is cirque groove on it. The check floating seal fitting is made up of columniform float and rail on the probe. The rail can prevent the float from falling off. On the float, there is a fixed round seal ring or in the probe there is a fixed cirque seal ring. The check floating seal fitting is made up of a protruding shaped float and rail which is on the probe and can prevent the float from falling off. On float protruding desk, securing a round seal ring or inside the probe securing a cirque seal ring. The float is solid or hollow. The stated valve is solenoid valve or electrical valve. operations are necessarily order dependent. In particular, these operations need not be performed in the order of presentation.

The phrase "in one embodiment" is used repeatedly. The phrase generally does not refer to the same embodiment, however, it may. The terms "comprising", "having" and "including" are synonymous, unless the context dictates otherwise.

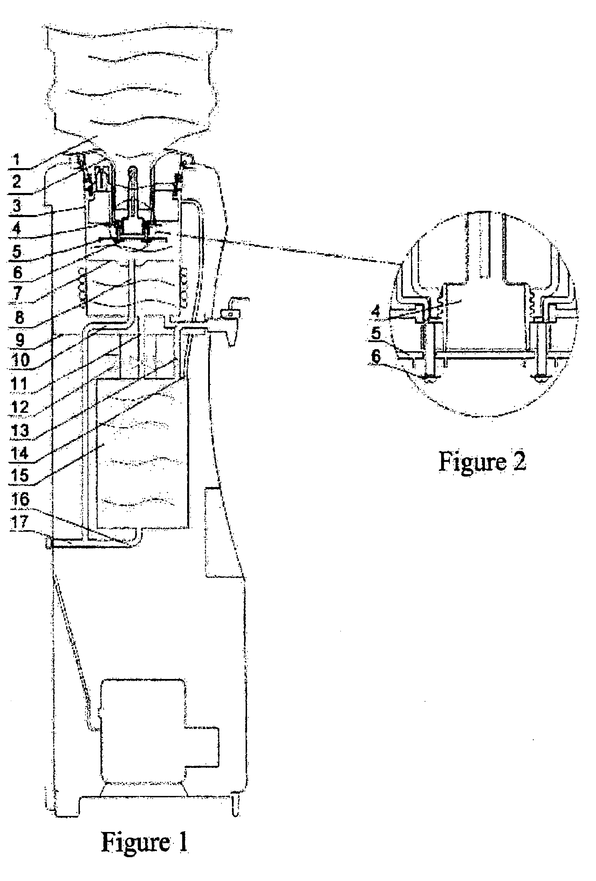

Now referring to Figure 1 and Figure 2 as in one embodiment of the present invention, shown is a side sectional view of a bottled water cooler and an exploded view of a particular area of the bottled water cooler . Shown is a cold tank 3 installed on the top of a cooler, inside of cold tank 3 there is bottle receptacle 2, and on the top of cold tank 3 there is a check valve structure which can efficiently prevent water from overflowing from the cooler due to a cracked water bottle 1. Inside of the groove of the stated bottled receptacle 2 is a probe 4 connecting with water bottle 1. Inside of the probe 4 is a check floating seal fitting. In this example, the stated unilateralism floating seal fitting is made up of floating board or disc 5 and screws 6 which can prevent floating board 5 from falling off and mat piece, circular ridge or seal. Floating disk 5 rest against the circular ridge. The area of contact between disk and ridge must be minimal to prevent molecular bond, that would cause the disk to hang up.

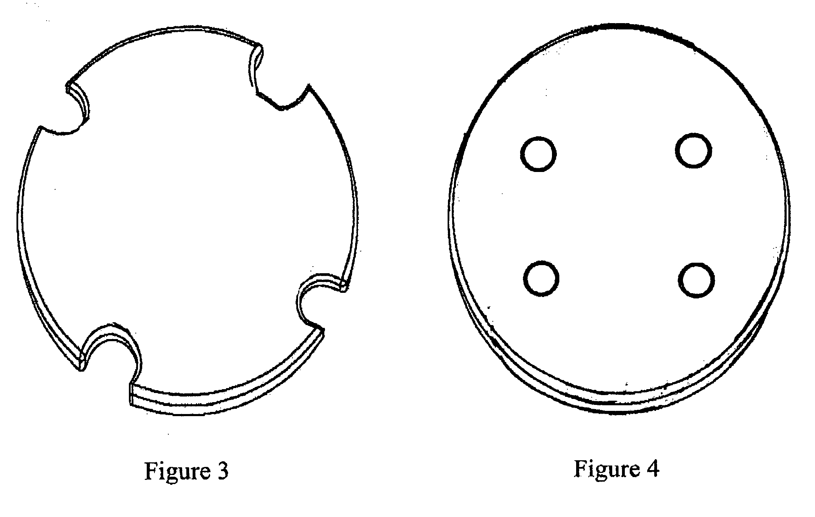

Referring to Figure 3 and Figure 4 as in one embodiment of the present invention, shown are top views of floating board 5. Floating board 5 is a thin disc shape made of polypropylene its specific gravity is 0.97 a little less then waters specific gravity, this way, floatage of floating board 5 is very small, so it is not fast when closing and opening in place. Polypropylene was chosen for two reasons: - specific gravity that makes it slow in closing and opening and high temperature resistance. Floating board 5 has no resistance when the opening place opens, it will not prevent water flowing fast when floating board 5 seals the opening place too fast due to big floatage. This allows floating board 5 in cold tank 3, the water level reached certain height to close the passage of probe 4, when water lever drop when using water in the cooler, floating board 5 move down due to gravity function, to open passage of probe 4, then water enter cold tank 3, till the water level reaches a certain height, the floating board 5 to close the passage of probe 4, this way it can prevent water in water bottle 1 and hot water in cold tank 3 converting, prevent water in water bottle 1 from heating therefore saving the water resource. Also, to avoid floating board 5 sticking with probe 4 due to molecule gravitation, floating board 5 must be bigger then the opening of probe 4, and make circular groove on the center part of floating board 5, it can reduce floating board 5 distorted due to high temperature, also provide sealing for flat surface on the edge of knife of the bottom of bottle receptacle 2. the stated cold tank 3 inside, and under probe 4, there is a baffle 7 through hollow baffle leader, baffle 7 separate cold tank 3 into 2 separate water level, this baffle leader end and hot tank 15 under cold tank 3 connect through cold and hot tank connecting tube 10, hot tank drain three-way pip 17 and hot tank inlet or outlet 16, cold water can flow to hot tank 15 through this passage from cold tank 3. between cold tank 3 and hot tank, there is a cold tank tube 11, and there is valve 12 on it, in this example the valve 12 is solenoid valve (the stated valve 12 also can be electrical valve), controlled by microprocessor to open and close. Usually when the solenoid close, to sterilize cold tank 3, the microprocessor will give signal to open solenoid, hot water enter cold tank 3 to sterilize with high temperature through cold and hot tank tube 11. between cold tank 3 and hot tank 15 there is a cold tank baffle 8, on top of the stated hot tank 15 there is a hot tank outlet tube 13 and a hot tank vent-pipe 14.

To sterilize as in one embodiment of the present invention under normal uses of the cooler cold tank 3 and hot tank 15 all have full water, and valve 12 is open; when there is a need to sterilize cold tank 3, the microprocessor refrigeration system close, and open valve 12, now using cold and hot water convection principles, the present invention makes water in cold tank 3 hot by convection in hot tank 15, hot water in the hot tank 15 enters cold tank 3 through tube 11, cold water in the cold tank 3 enters hot tank 15 through hot and cold tank tube, at the same time hot tank 15 heats the water whose temperature is low, circulate like this, till the temperature in cold tank 3 reaches 84C, then the microprocessor restarts the cold tank system and closes valve 12, cutting off the convection passage or tube 11. Now the cooler recovers to the normal state. For convenience we can set up times and periods to sterilize the cooler through the microprocessor.

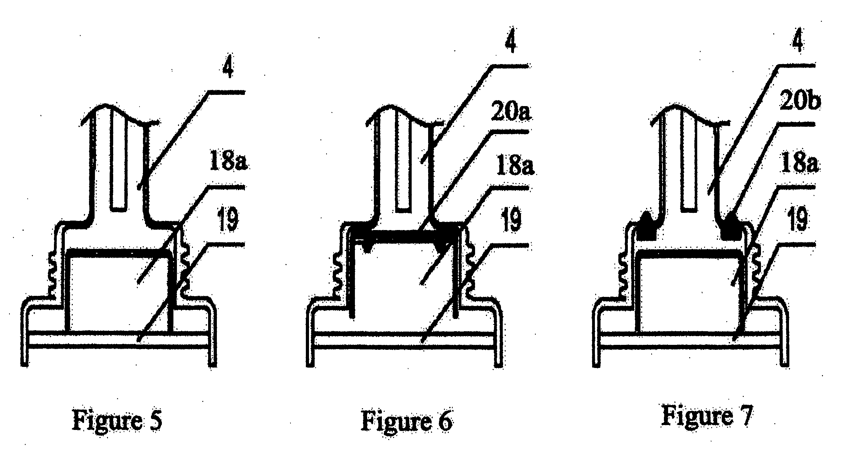

Referring to Figure 5 as in one embodiment of the present invention, shown is a check floating seal fitting is made up of float 18a, and rail 19 on the probe 4. The rail can prevent the float from falling off. Referring to Figure 6 as in one embodiment of the present invention, shown is a set a round seal ring 20a on float 18a. Referring to Figure 7 as in one embodiment of the present invention, shown is a fixed round seal ring 20b in the probe 4.

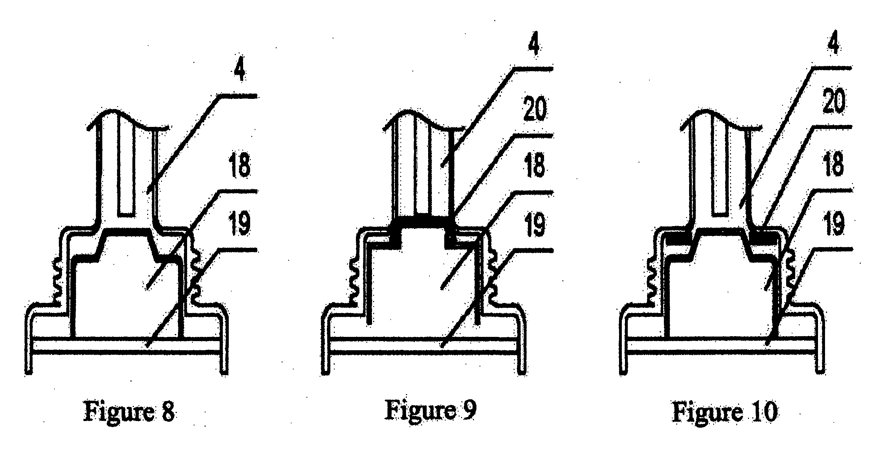

Referring to Figure 8 as in one embodiment of the present invention, shown is a the stated check floating seal fitting is a protruding shaped float 18b and rail 19 which is on the probe that prevents the float falling off Referring to Figure 9 as in one embodiment of the present invention, shown is a set seal ring 20c on protruding table of shaped float 18b. Referring to Figure 10 as in one embodiment of the present invention, shown is the probe 4 securing a circular seal ring 20d.

Float 18a and protruding shaped float 18d are made of food grade plastic which density is smaller than water and can be solid or hollow. The sterilization process in the same in all cases.

While the present invention has been related in terms of the foregoing embodiments, those skilled in the art will recognize that the invention is not limited to the embodiments depicted. The present invention can be practiced with modification and alteration within the spirit and scope of the appended claims. Thus, the description is to be regarded as illustrative instead of restrictive on the present invention.

## Claims

1. A bottled water cooler with hot water sterilizing system comprising:
a cold tank installed on a top of the cooler the cooler with a bottle receptacle and a bottle receptacle groove having a piercing core connecting with a water bottle, the cold tank has a baffle with a hollow baffle leader under the piercing core, one end of the baffle leader connects with a hot tank installed under the cold tank, the hot tank connecting tube connects to a draining three-way pipe and a hot tank inlet or draining pipe, a tank baffle is between the cold tank and the hot tank, the hot tank has a hot tank outlet, between the cold tank and hot tank there is a hot tank exhausting tube and a valve opening and closing the hot tank exhausting tube, inside of the probe there is a check floating seal fitting.

2. The bottled water cooler with hot water sterilizing system of claim 1 wherein the check floating seal fitting is made up of floating board and restricting bolts which can prevent the floating board from falling off and mat piece.

3. The bottled water cooler with hot water sterilizing system of claim 1 wherein the floating board is a thin disk shape and there is a circular groove on it.

4. The bottled water cooler with hot water sterilizing system of claim 1 wherein the check floating seal fitting is made up of float and rail on the probe, the rail can prevent the float from falling off.
